# NEW EUROPEAN PATENT SPECIFICATION

(11) **EP 1 656 215 B2**
(45) Date of publication and mention of the opposition decision: **25.12.2013**
(45) Mention of the grant of the patent: 29.09.2010
(21) Application number: 04764094.1
(22) Date of filing: 13.08.2004
(51) Int. Cl.: B05C 17/005, A61C 5/06, A61C 9/00, A61B 17/00, B01F 5/06, B01F 13/00, B01F 15/00, B05C 17/01

(54) **UNIT-DOSE SYRINGE FOR A MULTI-COMPONENT MATERIAL**
EINZELDOSISSPRITZE FÜR EIN MEHRKOMPONENTENMATERIAL
SERINGUE A DOSE UNITAIRE POUR PRODUIT A COMPOSANTS MULTIPLES

(30) Priority: 14.08.2003 DE 10337789
(43) Date of publication of application: 17.05.2006
(60) Divisional application: 07107819.0
(73) Proprietor: 3M Deutschland GmbH, 41453 Neuss (DE)
(72) Inventor: PEUKER, Marc, 86938 Schondorf (DE); HOHMANN, Arno, 81369 München (DE); PAUSER, Helmut, 86911 Diessen (DE)
(74) Representative: Vossius & Partner
(86) International application number: PCT/EP2004/009100
(87) International publication number: WO 2005/016170

(56) References cited:
- EP-A- 0 444 247
- EP-A- 0 815 802
- EP-A1- 0 157 121
- EP-A1- 0 261 466
- EP-A1- 0 289 882
- WO-A-03/041605
- DE-A1- 10 117 385
- DE-U1- 9 209 105
- GB-A- 2 086 248
- US-A- 3 587 982
- US-A- 3 738 535
- US-A- 4 538 920
- US-A- 4 676 657
- US-A- 4 690 306
- US-A- 5 743 436
- US-B1- 6 234 795
- US-B2- 6 572 031

## Description

### Background of the invention

### Technical field

The present invention relates to a unit-dose syringe comprising a static mixer for storage, mixing, and application of a multi-component material, preferably dental material.

### Description of the prior art

Known two-component syringes with static mixers have exchangeable mixing tips to enable multi-dose usage. Such syringes also become more and more available in small sizes for small amounts of material. Typically, small size syringes also comprise attachable/exchangeable tips - even if the material contained in the syringe is sufficient for single doses only. The reason for using attachable tips results from the usage of the same "closure system" for multi- and unit-dose syringes: all syringes are initially closed by caps which have to be replaced with the mixing tips prior to the application of the material.

Particularly for mnit-dose syringes a considerable part of the total costs results from the closure system described above because in addition to the disposable cap the interconnection between the syringe and the tip requires several movable parts within the mixing tip.

Furthermore, known syringes used as pre-filled syringes require elastic seals between the plunger and the cartridge as well as at the outlet end of the cartridge to ensure sufficient storage stability. Additional parts such as O-rings are typically used as seals. Those seals are made from rubber and are thus expensive. Furthermore, an additional sealing step is required during manufacturing of the syringe. Closure caps with or without seals are often used as an outlet seal, which have to be replaced with an application cannula by the dentist prior to the use of the syringe. Known approaches to eliminate seals did not result in sufficient storage stability. US-A-5 743 436 discloses a dispensing applicator for removably receiving a dual chamber cartridge. The cartridge has two side-by-side barrels each containing a chamber. The cartridge is connected to a housing that has an exit conduit. Within the exit conduit a static mixer may be received.

### Summary of the invention

It is the object underlying the present invention to provide an improved unit-dose syringe for multi-component material comprising a minimum number of parts which are easy to manufacture. This object is achieved with the features of the claims.

The present invention provides a unit-dose syringe providing an integrated and preferably self-opening closure to encapsulate material, preferably dental material, and to keep the compartments of the material separate during storage. The self-opening closure is easily openable for mixing and application of the material by moving preferably only one part of the syringe.

Furthermore, the cartridge of the syringe is preferably made of an elastic material, and the plunger is preferably made of a rigid material, thus providing a tight seal between the rigid plunger and the elastic, i.e. soft, cartridge.

According to a first aspect of the present invention, there is provided a unit-dose syringe according to claim 1.

Furthermore, the static mixer comprises a mixing helix, and an outlet tip at the front end of the mixing helix. According to a preferred embodiment, the outlet tip is connected to the mixing helix by a hinge. A collapsible static mixer is also encompassed by the present invention.

According to a preferred embodiment of the first aspect of the present invention, the outlet tip of the static mixer projects from the front end of the mixing tip when the static mixer is received in the mixing tip.

It is furthermore preferred that the outlet tip of the static mixer is accommodated within the mixing tip during storage of the syringe. In this case, the front end of the mixing tip and the outlet tip of the static mixer comprise corresponding retention means that allow the outlet tip to project beyond the front end of the mixing tip upon activation of the syringe, but prevent that the outlet tip completely extends beyond the mixing tip. The retention means at the front end of the mixing tip preferably comprises a recess in the wall of the mixing tip, and the retention means of the outlet tip comprises a projection at the circumference of the rear end of the outlet tip, wherein the projection is engagable by the recess once the syringe is activated and the outlet tip is moved outwards of the mixing tip.

According to an embodiment which belongs to the background of the invention, the mixing tip is connected to the cartridge by a hinge. This is advantageous because a fold-away mixing tip provides the option to mold the outlet tip integral with the mixing tip rather than with the mixer. As the mixing tip is tapered to the outlet end molding is only possible if the core can be removed from the back end of the mixing tip which is facilitated by this embodiment.

In an alternate embodiment of the first aspect of the present invention, the mixing tip forms an extension of a first compartment of the cartridge. In another embodiment belonging to the background of the invention the first and a second compartments are connected by a passageway provided adjacent the first end of the cartridge. The second compartment comprises a plug sealing the second compartment against that opening of the passageway facing towards the interior of the second compartment. The static mixer comprises at its rear end a plug sealing the first compartment against that opening of the passageway facing towards the interior of the first compartment. Thus, activation of the syringe by the plunger moves the two plugs along the longitudinal direction of the syringe in order to free the passageway so that material is allowed to flow from the two compartments into the mixing tip for mixing.

In another embodiment belonging to the background of the invention, a unit-dose syringe for a multi-component material comprises a cartridge having a first end and second end, and having a compartment for each component, a mixing tip being connectable with the cartridge at its first end and receiving a static mixer, and a plunger for dispensing material from the cartridge through the mixing tip, wherein the plunger is arranged at said second end of said cartridge.

According to this embodiment of the background of the invention, the cartridge preferably comprises a recess at its first end extending in the longitudinal direction for receiving the rear end of the mixing tip. The cartridge comprises radial openings in the wall of the recess for providing passageways from the compartments to the recess. Furthermore, the mixing tip comprises radial openings that correspond to and align with the radial openings in the recess wall to provide passageways from the compartments into the mixing tip.

Preferably, the static mixer comprises a mixing helix. Furthermore, the static mixer comprises a spacer at the rear end of the mixing helix. The spacer extends along the longitudinal axis of the static mixer. The said static mixer also comprises a closure element at the rear end of the spacer. The spacer extends in the longitudinal direction along the width of the passageways at the rear end of the mixing tip such that the closure element is located rearwards of the passageway openings.

In another embodiment forming background information, a unit-dose syringe for a multi-component material is provided, comprising a cartridge having a first end and a second end, and having a compartment for each component, wherein the compartments extend between the first end and the second end, a static mixer being integrally formed with the cartridge at the first end thereof, and a plunger for dispensing material from the cartridge. The plunger is arranged at the second end of the cartridge. Furthermore, a mixing tip connectable to the cartridge at the first end of the cartridge and receiving the static mixer is provided.

Each compartment of the cartridge preferably comprises outlet openings at the first end of the cartridge. In particular, the outlet openings of the compartments are directed along the longitudinal axis of the syringe.

It is furthermore preferred according to a first alternative of the embodiment forming background information that the mixing tip comprises an axially acting rotary slide valve at its end that is connectable to the first end of the cartridge. The axially acting rotary slide valve preferably comprises passageways and seal areas that are alternately alignable with the outlet openings of the cartridge compartments. More preferably, the valve also comprises a locking mechanism that is engageable with a corresponding locking mechanism at the first end of the cartridge. The locking mechanism at the cartridge comprises pins that are engageable in corresponding recesses forming the locking mechanism of the valve. Preferably, the pins and said recesses are formed such that a thread lock is obtained interlocking the mixing tip and the cartridge in the longitudinal direction of the syringe.

According to a second alternative of the embodiment forming background information, the outlet openings of the compartments are directed transverse to the longitudinal axis of the syringe. In this case, the mixing tip comprises a radially acting rotary slide valve at its end that is connectable to the first end of the cartridge. The radially acting rotary slide valve preferably comprises a body member forming a cavity that corresponds to the outer surface of the cartridge in the area of its first end for receiving the first end of the cartridge. The wall of the cavity comprises recesses along the longitudinal axis of the body member, and the recesses are alignable with the outlet openings of the cartridge for forming passageways from the compartments of the cartridge to the static mixer. The use of a radially acting rotary valve in combination with transversely directed outlet openings is advantageous for optimum sealing of the cartridge especially for low viscosity materials.

According to a preferred embodiment of all aspects of the present invention, the cartridge comprises at its outer surface extensions or protrusions to provide the cartridge with a substantially circular circumferential outer surface.

It is also a preferred feature that the cartridge has a rounded circumferential surface, and comprises at least one internal separation wall. Alternatively or preferably, the compartments are arranged concentrically.

It is also preferred in all aspects of the present invention that the plunger comprises a separate piston for each compartment of said cartridge.

The cartridge is preferably made from an elastic material, more preferably from a thermoplastic elastomer. The plunger is preferably made from a rigid material.

In another embodiment forming background information, a unit-dose syringe for a multi-component material is provided that comprises a cartridge having a first end and a second end, and has a compartment for each component. The compartments extend between said first end and said second end. A plunger for dispensing material from the cartridge is arranged at a second end of the cartridge. Furthermore, a mixing tip is connectable to the cartridge at the first end of the cartridge for receiving a static mixer.

In this embodiment forming background information, the syringe comprises two compartments. A first compartment of the cartridge comprises an opening at the first end of the cartridge. Preferably, the first compartment and a second compartment are rotatable relative to each other.

According to a preferred embodiment forming background information, the wall of the first compartment comprises a first channel being inclined with regard to the longitudinal axis of the syringe, and the wall of the second compartment comprises a second channel also being inclined with regard to the longitudinal axis of said syringe. Rotational movement of the first compartment relative to the second compartment brings the first inclined channel and the second inclined channel into alignment to provide a passageway from the first to the second compartment.

According to a second aspect of the present invention a unit-dose syringe for a multi-component material being pre-filled with a multi-component dental material is provided.

The unit-dose syringe of the present invention is advantageous in that it consists of three components only, i.e., a cartridge with mixing tip, static mixer, and plunger, or, alternatively, a cartridge with static mixer, mixing tip, and plunger. This is more economic compared to the prior art because less components are to be manufactured, and all parts can be assembled easily. Additional gluing or welding steps are not necessary.

### Brief description of the drawings

- Fig. 1: shows an assembled unit-dose syringe according to the first aspect of the present invention;
- Fig. 2: shows the multi-component cartridge with an integrally connected mixing tip of the unit-dose syringe of Fig. 1;
- Fig. 3: shows the plunger of the unit-dose syringe of Fig. 1;
- Fig. 4: shows the static mixer of the unit-dose syringe of Fig. 1, having an outlet tip and closure plugs;
- Fig. 5: shows the static mixer of the unit-dose syringe of Fig. 1, having an alternative outlet tip hinged to the mixer, and closure plugs;
- Fig. 6: shows an assembled unit-dose syringe in schematic form, and particularly shows a retention mechanism; in the upper drawing, the syringe is shown in its inactivated state, whereas the lower drawing shows the activated syringe with engaged retention mechanism;
- Fig. 7: shows in schematic form an outlet opening of the cartridge being closed by a closure cap;
- Fig. 8: shows how the closure cap of Fig. 7 is removed due to the applied pressure;
- Fig. 9: shows in more detail the retention mechanism at the front end of the mixing tip; the upper drawing shows the inactivated state, and the lower drawing shows the activated state;
- Fig. 10: shows an embodiment of the background of the invention with regard to the attachment of the mixing tip to the cartridge;
- Fig. 11: shows an alternative closure of an embodiment of the background of the invention, i.e., a foil closure for the outlet openings of the cartridge, which could be peeled away or punched by appropriate elements at the rear end of the mixing tip;
- Fig. 12: shows an alternative preferred embodiment of the unit-dose syringe, having a cartridge with an outer round shape and an internal separation wall;
- Fig. 13: shows an alternative embodiment of the background of the invention of the unit-dose syringe in its inactivated state, wherein the mixing tip forms an extension of one of the cartridge compartments;
- Fig. 14: shows the unit-dose syringe of Fig. 13 in its activated state;
- Fig. 15: shows an alternative embodiment of the syringe of Figs. 13 and 14 with a collapsible mixing helix;
- Fig. 16: shows a further preferred detail of the unit-dose syringe of the background of the invention;
- Fig. 17: shows an alternative opening mechanism of the syringe according to Fig. 16;
- Fig. 18: shows a further modified alternative of the syringe of Fig. 16;
- Fig. 19: shows a further modified alternative of the syringe of Fig. 16;
- Fig. 20: shows an embodiment of the background of the invention of the syringe having a recess at its front end for receiving an exchangeable mixing tip;
- Fig. 21: shows the exchangeable mixing tip of Fig. 20 in greater detail, being received in the recess of the cartridge;
- Fig. 22: shows the mixing helix of Fig. 21 in more detail;
- Fig. 23: shows a schematic of a self opening valve for closing the outlet of the cartridge;
- Fig. 24: shows an alternative embodiment of the background of the invention suitable for the mixing of powder/liquid compositions;
- Fig. 25: shows in more detail a preferred opening mechanism for a powder/liquid syringe; the left drawing shows the mechanism in its activated state;
- Fig. 26: shows an assembled unit-dose syringe according to another embodiment forming background information;
- Fig. 27: shows the syringe of Fig. 26 without the mixing tip;
- Fig. 28: shows the correction between the cartridge and the mixing tip of the syringe of Fig. 26 in greater detail; the left drawing shows the inactivated state whereas the right drawing shows the activated state;
- Fig. 29: shows the passageways of the syringe of Fig. 26 for the material flow after the syringe has been opened as well as the sealing areas sealing the during storage;
- Fig. 30: the lock system of the syringe of Fig.26, locking the mixing tip to the cartridge in detail; the left drawing shows the closed position;
- Fig. 31: shows a grip support as a preferred feature of the cartridge;
- Fig. 32: an alternative embodiment of the embodiment forming background information, using a rotary slide valve as opening mechanism; ,
- Fig. 33: shows a cross-sectional view of the opening mechanism of Fig. 32;
- Fig. 34: shows the front end of the cartridge with radial outlet bores, for use in a syringe according to Fig. 32;
- Fig. 35: shows three cross-sectional views of the closure/opening of the radial bores of Fig. 34 by the mixing tip;
- Fig. 36: shows a preferred design of the opening/closing valve of the syringe of Fig. 32; and
- Fig. 37.: shows an alternative grip support at the cartridge.

### Detailed description of preferred embodiments of the present invention

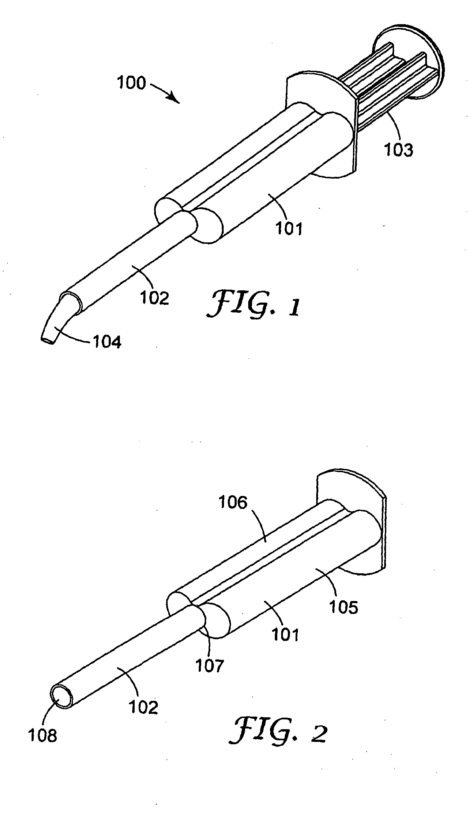

Fig. 1 shows an assembled unit-dose syringe 100 according to the first aspect of the present invention. The assembled syringe 100 comprises a cartridge 101 with a mixing tip or mixing orifice, respectively, 102 being integrally formed with the cartridge 101, and a plunger 103. The multi-component cartridge 101 which is shown separately in Fig. 2 comprises a first compartment 105 for receiving a first component of the material to be mixed, and a second compartment 106 for receiving a second component of the material to be mixed. Within the cartridge 101, the two compartments are separated from each other by a separation wall, for example, in order to avoid that the two components mix with each other prior to the use of the syringe and that the two components undergo an undesired reaction. The mixing tip 102 is connected to the cartridge 101 at its front end 107 and itself comprises a front end opening 108.

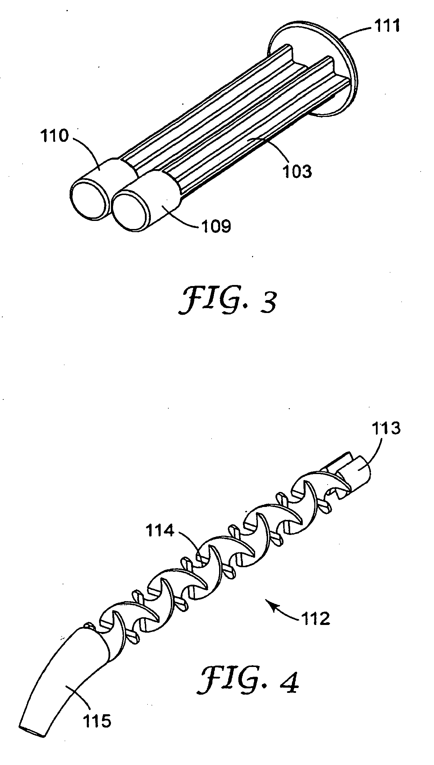

The plunger 103 which is shown in more detail in Fig. 3 comprises two pistons, 109, 110 which are integrally connected with each other at the rear end 111 of the plunger 103. The two pistons 109, 110 engage in each of the two compartments or barrels 105, 106 of the cartridge 101, in order to dispense the components contained in the compartments 105, 106. In the inactivated state of the syringe, the pistons 109, 110 close the barrels 105, 106 of the cartridge on a first end, i.e., the rear end.

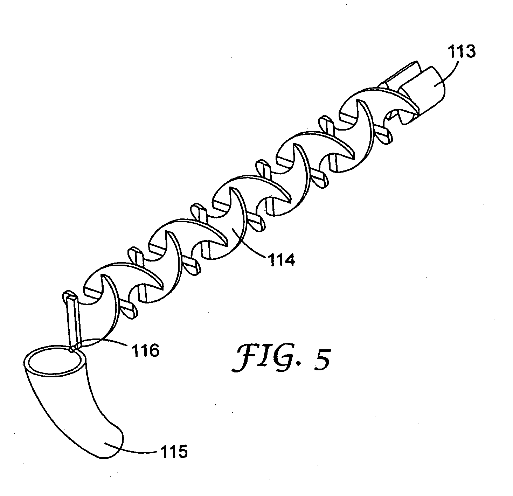

The syringe of the first aspect of the present invention further comprises a static mixer 112 which is received in the mixing tip 102 or mixing orifice, respectively. At its rear end, the static mixer 112 comprises closure plugs 113 which close each of the outlet openings of the barrels 105, 106, i.e., the front end openings of the cartridge 101. Thus, the compartments are kept encapsulated and separated during storage. The static mixer 112 further comprises a mixing helix 114 and an outlet tip 115. The integrally formed outlet tip 115 is shown in Fig. 4. For easier moulding, the outlet tip 115 can be fixed to the mixing helix 114 with a hinge construction 116 as shown in Fig. 5. Such a structure is advantageous if the mixing helix 114 has to be assembled from the front end of the mixing barrel 102.

The syringe 100 according to the first aspect of the present invention is activated by moving the plunger 103 with its pistons at its front end into the cartridge 101. This causes the plugs 113 formed at the static mixer 112 to be pushed out of the front outlet openings of the compartments of the cartridge 101 due to hydraulic pressure of the dental material (paste). The mixing helix 114 is thus enabled to be moved along the longitudinal axis of the mixing tip 102 within a distance defined by the contractibility/elasticity of the mixing helix 114. Alternatively the mixing helix 114 can move towards the outlet of the mixing tip 102 over a defined distance. The distance the mixing helix 114 can move may be limited by a retension mechanism 118, 119 inside of the mixing tip 102, e. g. a step (reduction) in diameter.

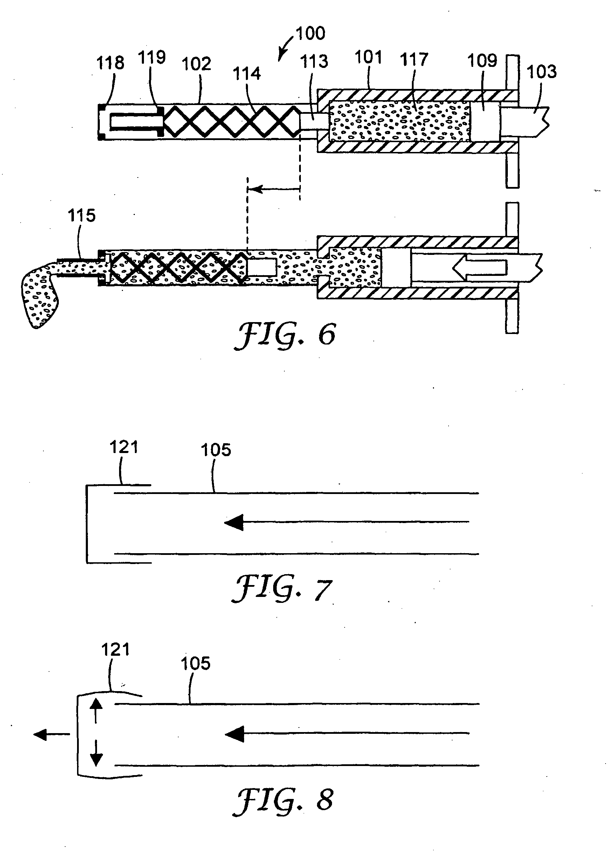

This operation is illustrated in the two drawings of Fig. 6. The upper drawing shows the syringe 100 in its inactivated state, whereas the lower drawing of Fig. 6 shows the syringe 100 during activation. Upon movement of the plunger 103, the piston 109 urges the paste 107 in the cartridge 101 against the plug 113 which closes the outlet opening of the cartridge 101. Once the pressure is high enough, the plugs 113 are pushed out of the outlet opening, and the pastes 117 flow through the mixing helix 114, are mixed, and finally dispensed from the outlet tip 115. Since the plugs 113 are smaller than the inner diameter of the mixing tip 102, the pasty materials can flow around the plugs and through the mixing helix 114 of the static mixer 112.

In addition to illustrating the general operation of the syringe 100, Fig. 6 shows further preferred features. First, according to the preferred embodiment of Fig. 6, the outlet tip 115 does not project beyond the outlet opening of the mixing tip 102 in the inactivated state of the syringe 100 but is accommodated within the mixing tip 102. Only upon activation of the syringe 100, the outlet tip 115 is pushed out of the front opening of the mixing tip 102 and then projects beyond the mixing tip 102. Secondly, a further preferred feature is the provision of a retention mechanism 118, 119. Such a retention mechanism 118, 119 is preferred for front-assembled syringes. The retention mechanism is provided at the opening of the mixing tip (118) as well as at the rear end of the outlet tip 115 (119). Thus, forward movement of the outlet tip 115 is stopped by the retention mechanism which at the same time forms a seal between the outer surface of the outlet tip 115 and the inner surface of the mixing tip 102 thus ensuring that the mixed paste flows through the outlet tip 115.

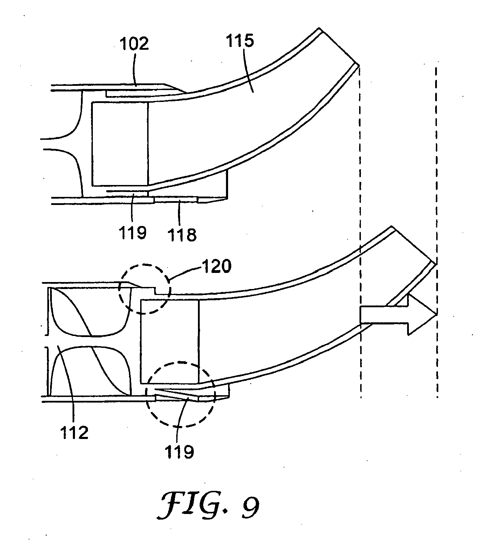

One such retention mechanism is shown in greater detail in Fig. 9. In the upper drawing of Fig. 9, the outlet tip 115 already slightly projects beyond the mixing tip 102, whereas in the lower drawing the outlet tip 115 is in its fully projected position. The part 119 of the retention mechanism provided at the outlet tip 115 is in the form of a projecting flange, whereas the part 118 of the retention mechanism provided at the mixing tip 102 is in the form of a recess. Once the flange 119 has reached the recess, it springs out into the recess and prevents further movement of the static mixer 112. At the same time, a seal 120 is formed between the outlet tip 115 and the mixing tip 102.

Figs. 7 and 8 illustrate an alternative closure mechanism for the compartments 105, 106. Instead of the plugs 113 of the static mixer 112, flexible caps 121 are used as a closure for the barrels. Upon application of pressure by the plunger 103, the caps are widened due to their flexibility, and the pressure would easily slide the caps from the barrels. The arrows in Fig. 8 illustrate how the forces caused by the applied pressure would act against the walls of the flexible cap.

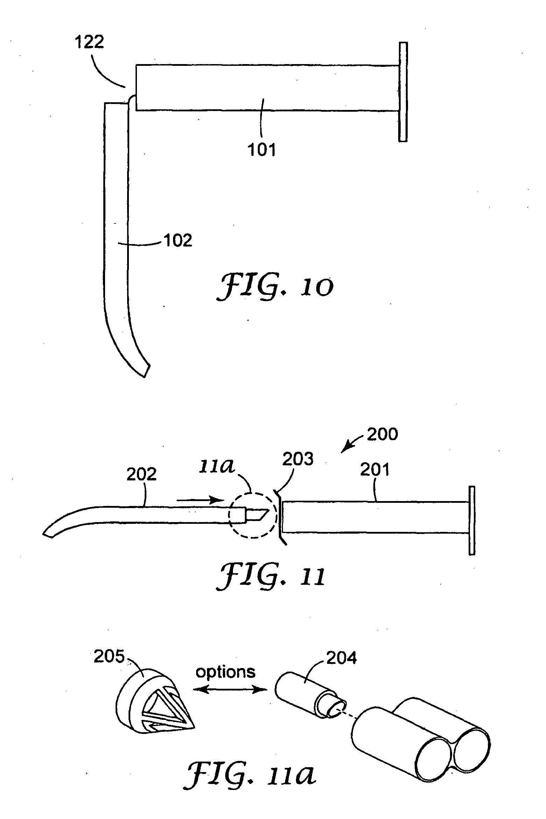

A further alternative embodiment belonging to the background of the invention is shown in Fig. 10. According to this embodiment, the outlet tip is an integral part of the mixing tip 102 and not of the static mixer 112. In order to enable the moulding of this embodiment, the mixing tip 102 is preferably connected to the cartridge 101 via a film hinge 122.

As an alternative option, the mixing barrel is fixed at the cartridge via plug connections. In this embodiment, the mixing barrel would be separate from the cartridge, and either the mixing barrel or the cartridge comprise interconnection sockets for assembly with the other part, respectively. This alternative embodiment also belonging to the background of the invention is shown in Fig. 11.

The syringe assembly 200 of Fig. 11 comprises a cartridge 201 and a separate mixing tip/mixing barrel 202. The front openings of the two compartments of the cartridge 201 are closed with foil-type closures 203 which improves the storage stability properties. The foil-type closure 203 is preferably made as a peel closure. Alternatively, the foil-type closure is punchable. This option is illustrated in Fig. 11. The mixing barrel 202 comprises interconnecting sockets 204 or 205 with punching elements at its rear end, as shown in Fig. 11a.

The use of a fold away mixing barrel as shown in Fig. 10 or of a separate mixing barrel is advantageous because such an arrangement saves space due to the reduced overall length of the syringe during storage.

All the embodiments described so far preferably comprise a kind of retention mechanism for locking the mixing barrel to the cartridge after assembly (snap in solutions).

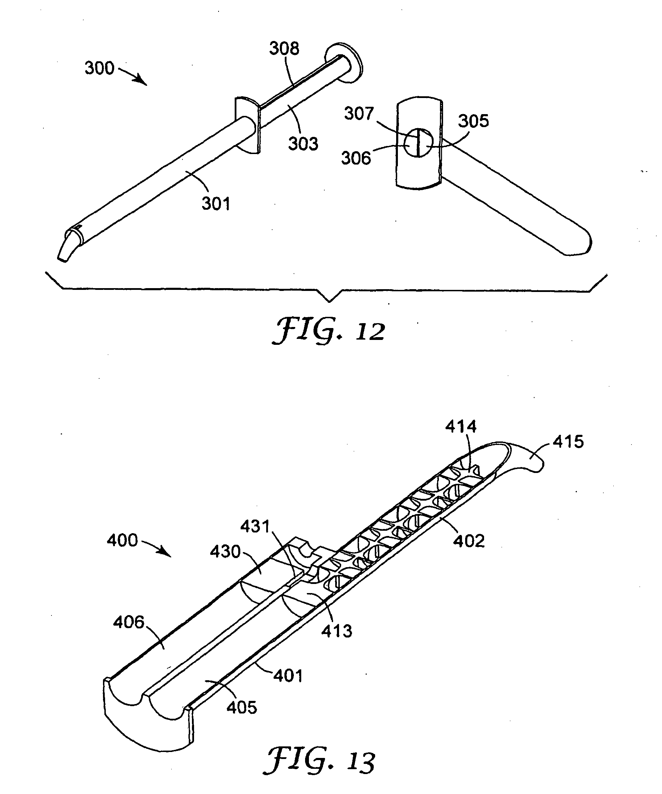

Fig. 12 shows a further alternative embodiment. Syringe 300 comprises a cartridge 301 which has an outer round shape, preferably cylindrical, and at least one inner separation wall 307 separating two compartments 305, 306 from each other. A plunger 303 is accordingly adapted and particularly comprises a longitudinal slot 308 for receiving the separation wall 307 during use of the syringe 300. As an alternative option (not shown), the compartments are arranged concentrically.

A further syringe 400 belonging to the background of the invention is shown in Figs. 13 to 15. In this embodiment, syringe 400 comprises a cartridge 401 being formed of an elongated main barrel 405 and one or more side barrels 406. The main barrel 405 also forms the mixing barrel 403, preferably comprising an intra-oral outlet tip 415. As an alternative option, the barrels are arranged concentrically (not shown). Within the mixing barrel 403, a mixing helix 414 is provided. Rear plug 413 closes the front end of the main barrel 405. At the front end of the side barrel 406, a piston 430 is provided that closes a passageway 431 connecting the main with the side barrels. In the inactivated state of the syringe 400, plug 413 closes the main barrel 405 such that the material contained in the main barrel 405 cannot reach the passageway 431, i.e., plug 413 separates the material in main barrel 405 from the passageway 431.

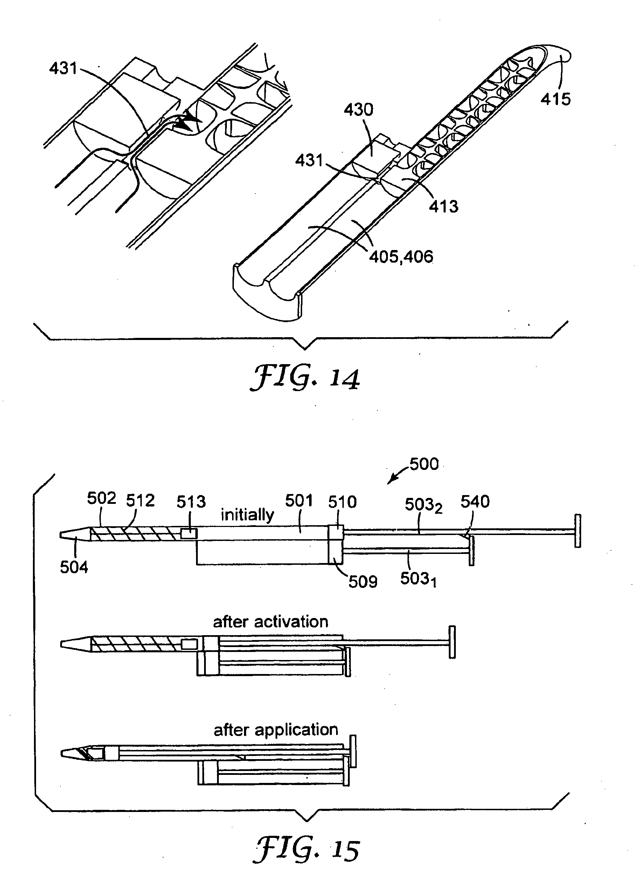

For activation of the syringe, a dual plunger (not shown) is moved into the syringe 400. This causes the piston 430 within the side barrel 406 and at the same time the mixing helix 414 with its plug 413 to move forward and to open the passageway 431, which allows the material in the two compartments 405, 406 to flow through the passageway 431 into the mixing barrel 403. This is illustrated in Fig. 14.

In an embodiment belonging to the background of the invention, as shown in Fig. 15, a collapsible mixing helix 512 is used. In this case, the arrangement and structure of the pistons 509 and 510 and plungers is different to the previously described embodiments. In this embodiment, two plungers 503₁ and 503₂ are provided. One of these two plungers is shorter (plunger 503₁) than the other. The longer plunger 503₂ comprises a projection 540 that interacts with the other plunger 503₁ such that movement of the shorter plunger in longitudinal direction, i.e. into the cartridge 501 also causes movement of the other, longer plunger. Projection 540 functions as a catch. As shown in the drawing in the middle of Fig. 15, after activation of the syringe 500, the two pistons have reached the front end of the cartridge 501 and have pressed the components of the material to be mixed into and through the collapsible mixing helix 512. In order to also remove and use the material that remains in the mixing tip 502, the longer plunger 503₂ is pushable further until it reaches the outlet tip 504. This is illustrated in the bottom drawing of Fig. 15. In this state, i.e., after application, the mixing helix is substantially completely collapsed within the outlet tip 504.

As an option, the passageway for the material flow can be split into passageways for each component. Furthermore, the passageways can be arranged in different length positions within the barrel in order to cause one or more components to flow over prior to others.

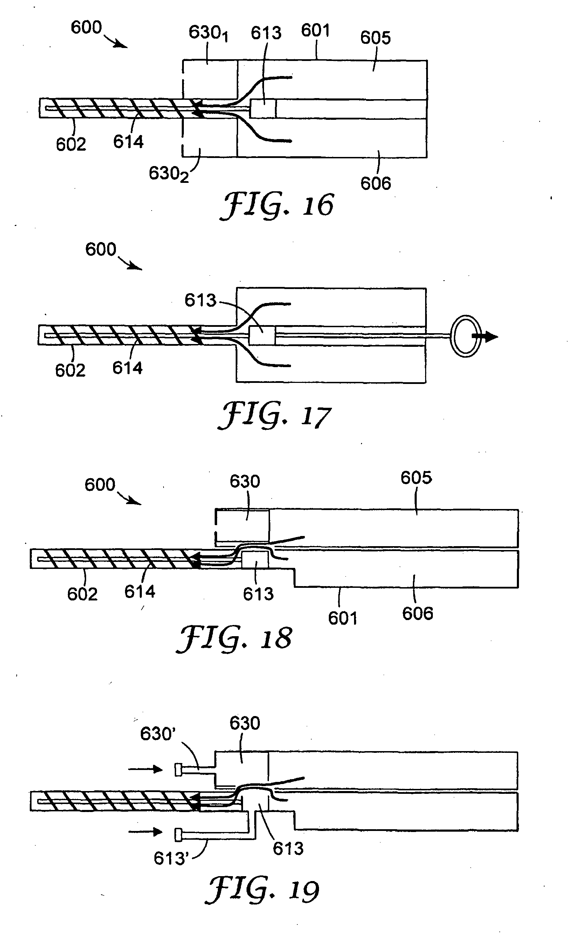

In a further preferred embodiment of the background of the invention, the main barrel of the cartridge is used as a mixing barrel only, i.e. no material is stored in the main barrel. In this case, the material components are arranged in separate barrels around the mixing barrel (i.e., as separate barrels, or concentrically). The mixing helix is then arranged in the syringe as shown in Fig. 16. In the embodiment shown in Fig. 16, two plugs 630₁ and 630₂ are provided at the front end of the material barrels 605, 606. During storage of the syringe 600, these two plugs close the passageway from the barrels into the mixing tip 602. In Fig. 16, however, the plugs have already been moved to the front end of the cartridge 601 (due to the pressure applied by the external pistons), thus opening the passageways so that the components of the material to be mixed can flow into the mixing tip 602, as indicated in Fig. 16 by the arrows. Plug 613 closes the mixing barrel in the back end direction.

This construction is advantageous if the required diameter for the mixing helix 614 is much smaller than the diameter (or cross-section) of the smallest material barrel because the remaining material within the mixing barrel can thus be reduced to a minimum. This construction is also usable with a collapsible mixer 614. In this case a separate piston for compressing the mixer wound be used. This piston - placed within the rear end of the mixing barrel - would be movable independently from the material applicator pistons.

An alternative opening mechanism is illustrated in Fig. 17. In this embodiment of the background of the invention, the plugs 630₁ and 630₂ are not necessary. Opening is made by pulling the mixing helix 614 with the closure plug 613 backwards. Thus, the passageways for the material components are opened, as indicated by the arrows.

A further modified version of this embodiment of the background of the invention is shown in Fig. 18. In this alternative the mixing barrel is a tapered part of one of the barrels 605, 606 containing material. The function of this alternative is similar to the embodiment shown in Fig. 14. Upon application of pressure by the piston(s), plug 630 is moved forwards and opens a passageway for the material contained in barrel 605. Furthermore, plug 613 is moved such that the material stored in barrel 606 can also flow through this passageway to be mixed with the other component in the mixing helix 614 in mixing tip 602.

As shown in Fig. 19, plugs 630 and 613 preferably comprise extensions 630' and 613', respectively, for example to close the passageway again.

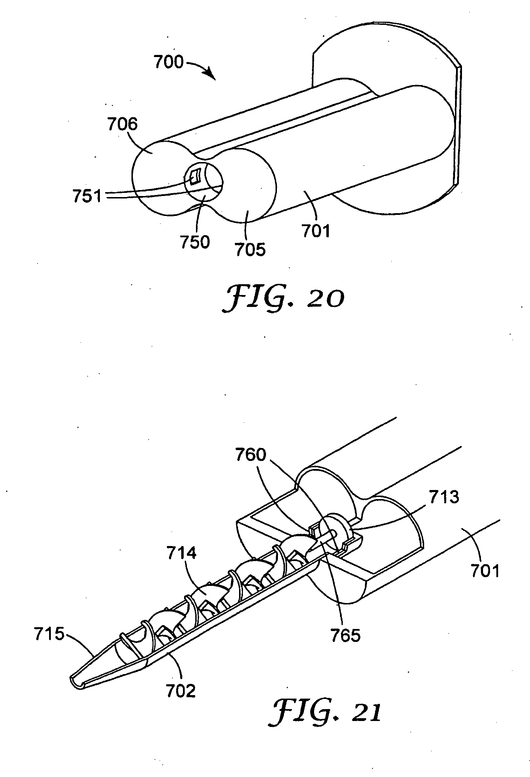

According to another embodiment of the background of the invention, and as shown in Fig. 20, cartridge 701 of syringe 700 comprises a recess 750 at its front end, e.g., a cylindrical bore, for receiving an exchangeable mixing tip/mixing barrel. The recess 750 comprises lateral passageways 751 into the material barrels 705, 706. The exchangeable mixing barrel also comprises passageways which can be aligned to the passageways 751 in the recess 750 thus forming a passageway from the material barrels to the inside of the mixing tip.

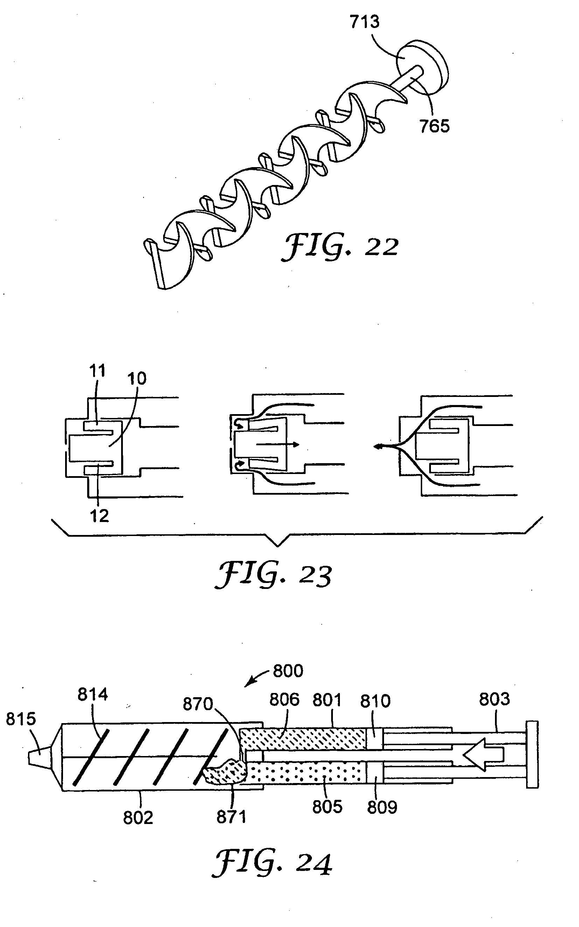

Fig. 21 shows the embodiment of Fig. 20 in more detail with a mixing tip 702 inserted into the recess 750. The mixing tip 702 comprises an outlet tip 715 and a mixing helix 714. At the rear end of the mixing tip 702, substantially opposite throughholes 760 are provided to form passageways connecting the interior of the cartridge 701, i.e., the material barrels with the interior of the mixing tip 702. The rear end of the mixing helix 714 comprises plug 713 that forms a closure of the rear end of the mixing tip 702. Upon activation of the syringe 700, the components contained in the material barrels flow through the passageways into the mixing tip 702 where they are mixed homogeneously by the mixing helix 714. In the area of the passageways, the mixing helix 714 comprises an inlet spacer 765 to allow the material to easily flow into the mixing tip 702. This is shown in more detail in Fig. 22.

This embodiment of the background of the invention is advantageous because no forces occur in the longitudinal direction between the cartridge an the mixing tip resulting from the material flow through lateral passageways. Even forces in the longitudinal direction between the mixing tip 702 and the mixing helix 714 are eliminated as the closure plug 713 of the helix compensates for the forces resulting from the material flow.

An additional advantage is that the connecting system used in this embodiment can be used as a valve and provides the possibility to use the syringe as multi-dose system. In this case, the mixing barrel is rotated within the recess until the passageways in the mixing tip and the passageways in the recess, respectively, are unaligned, thus closing the cartridge. A separate cap for closing the cartridge during storage is therefore not necessary. The risk of using a wrong mixer with the material filled in the cartridge is reduced.

The option of having a self-opening valve at the front end of the cartridge is described in more detail with regard to Fig. 23. Fig. 23 shows in its left drawing a plug 10, preferably cylindrical, that comprises a concentric lip I 1 projecting along a substantial part of the length of the plug so that a concentric U-shaped recess 12 is formed. Upon application of pressure, as shown in the middle drawing of Fig. 23, the flexible lip is deformed, and subsequently pushed backwards so that a passageway into the mixing tip (not shown) is opened (see arrows in the right drawing of Fig. 23).

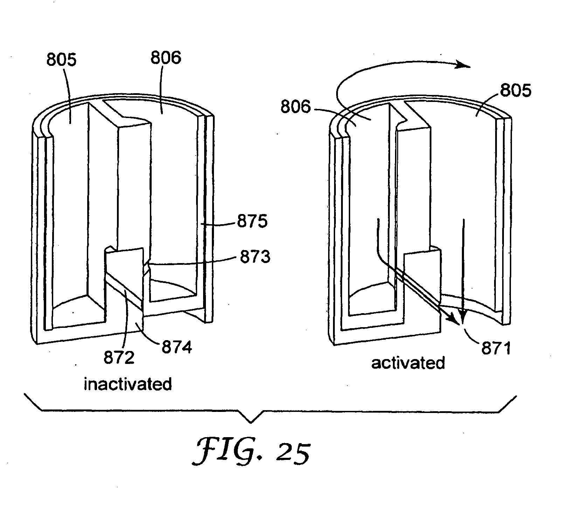

The aspects and embodiments described above are particularly useful for the mixing of paste/paste compositions. In the following, an embodiment belonging to the background of the invention, for powder/liquid compositions will be described with reference to Figs. 24 and 25.

According to the embodiment shown in Fig. 24, mixing of powder and liquid can be achieved if the liquid chamber 806 of the cartridge 801 is closed on the outlet side. During activation of the syringe 800, the liquid is forced to flow through a small side channel 870 into the powder chamber 805 which is open on the outlet side 871. Due to the fluid flow, the powder will be carried out of the cartridge 801 into the mixing barrel 802 while powder and fluid are pushed consistently by the plunger 803 with pistons 809, 810, respectively. The pre-mixed powderliquid-mix is homogeneously mixed by the mixing helix 814, and finally dispensed through outlet tip 815.

Such a construction would also work with a collapsible mixing helix.

A preferred opening mechanism for a powder/liquid syringe 800 is shown in Fig. 25. For ease of explanation, Fig. 25 merely shows a partial cross-sectional view of the two barrels of the cartridge, i.e., the barrel 805 for the powder, and the barrel 806 for the liquid. The arrangement as shown in Fig. 25 provides a double function rotary slide valve. In the inactivated position as shown in the left drawing of Fig. 25, the two barrels 805, 806 are separated from each other by the surrounding walls 874 and 875. Inclined channels 872, 873 are provided in the walls, which are unaligned in the inactivated state. In this embodiment, the two barrels are rotatable relative to each other, as indicated by the top arrow in the right drawing of Fig. 25. For activation, the inner barrel 806 with its wall 875 is rotated by 180° until the inclined channel 873 provided in wall 875 is aligned with the other inclined channel 872 provided in wall 874. The liquid flows through the resulting inclined channel At the same time, the front opening 871 is opened thus allowing the liquid/powder mixture to flow out, and into the mixing tip (not shown).

Another embodiment forming background information will now be described. According to this aspect, the syringe is formed by the integral combination of cartridge and static mixer.

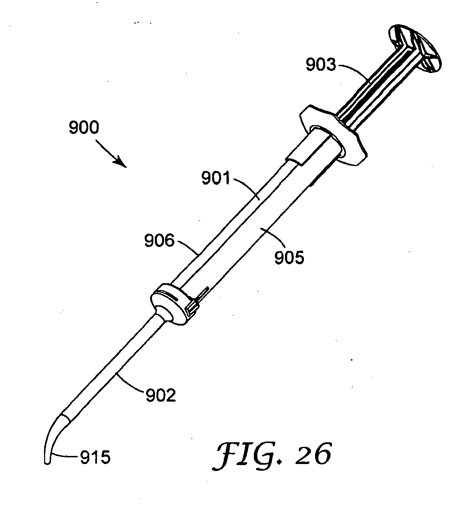

According to Fig. 26, syringe 900 comprises a cartridge 901 having compartments or barrels 905, 906 for storing the components of the material to be mixed. A plunger 903 is provided, comparable to the plungers described in the context of the above aspects and embodiments. At the front end of the cartridge 901, a mixing tip 902 with outlet tip 915 is attached. The syringe 900 incorporates a static mixer, i.e., the mixing helix as an integral part.

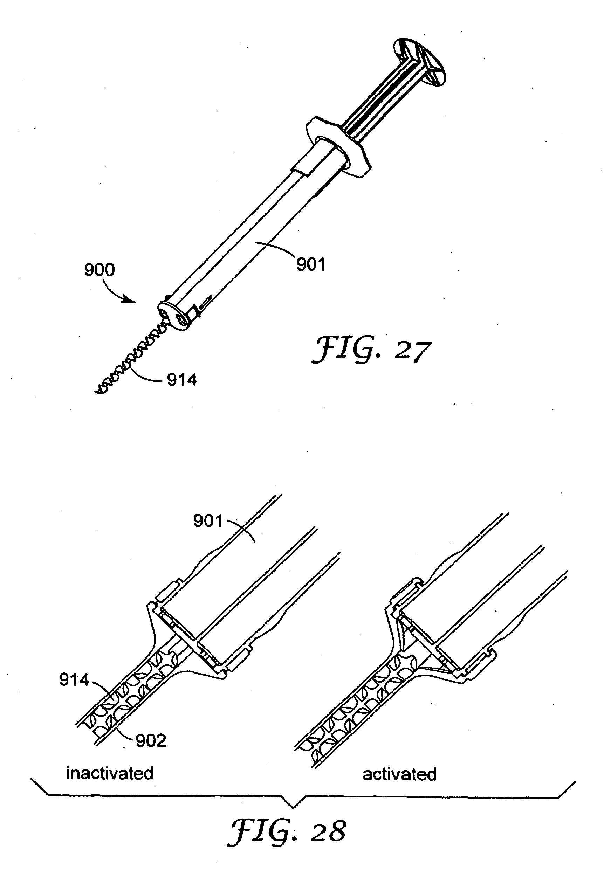

Fig. 27 shows the same syringe 900 without the mixing tip 912. Fig. 27 clearly illustrates that the mixing helix 814 is an integral part of the cartridge 901.

Fig. 28 shows a partial cross-sectional view of syringe 900 of Fig. 26. In Fig. 28, the mixing tip 902 is attached to the cartridge and covers the mixing helix 914. In the embodiment shown in Fig. 28, a rotary valve is used as an opening mechanism. In the left drawing of Fig. 28, the rotary valve is closed (inactivated state), and in the right drawing of Fig. 28, material flow is enabled as indicated by the arrows (activated state).

The rotary valve is designed such that pastes can flow through the cartridge outlets essentially parallel to the longitudinal axis of the cartridge. This prevents increased extrusion forces caused by changes of the flow direction. The syringe 900 is opened by rotating the intra-oral outlet tip by a predetermined angle, for example 90°.

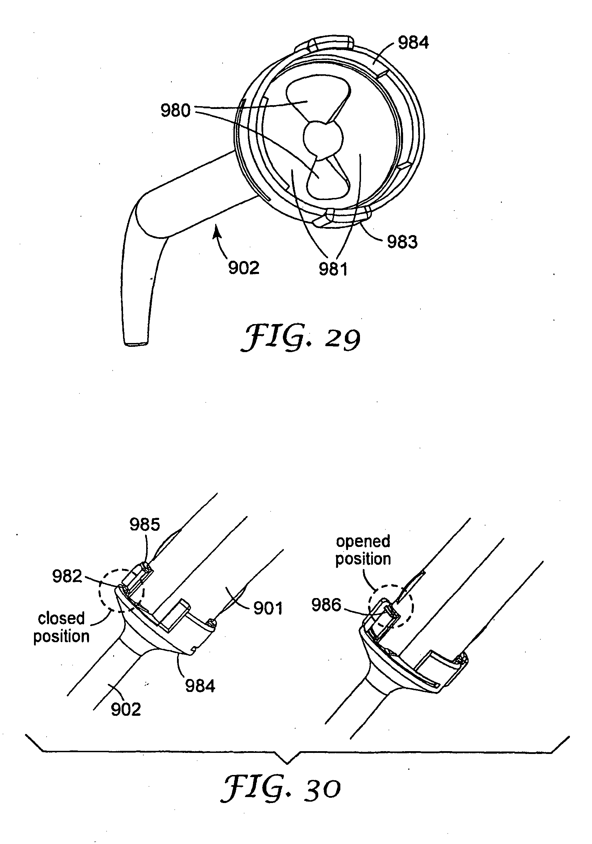

The passageways 980 of the rear end of the mixing tip 902 to be attached to the cartridge are shown in more detail in Fig. 29. The passageways are substantially triangularly shaped, and between the two passageways 980 there are provided two sealing areas 981 that seal the openings in the front end of the cartridge during storage. Rotation of the rotary valve aligns the passageways 980 with the openings in the cartridge.

According to a further preferred embodiment, as shown in Fig. 30, a twist lock system is provided that enables the mixing tip 902 to be assembled easily to the cartridge while disassembly is impossible or at least substantially prevented. The syringe is closed by rotating the mixing tip to a closed position. Pins 982 at the cartridge engaging in recesses 983 within the coupling part of the mixing tip 902 are acting as a thread in order to cause a sealing pressure in a longitudinal axis of the syringe. The left drawing of Fig. 30 shows a partially broken away part of the rear end of the mixing tip 902 forming an engagement flange 984. Second pins 985 at the cartridge engaging in second recesses 986 in the flange 984 of the mixing tip 902 act as a safety lock after the syringe has been opened by rotation of the tip in an opened position. This safety lock prevents the tip from being pushed off by the extrusion forces during dispensing material from the syringe.

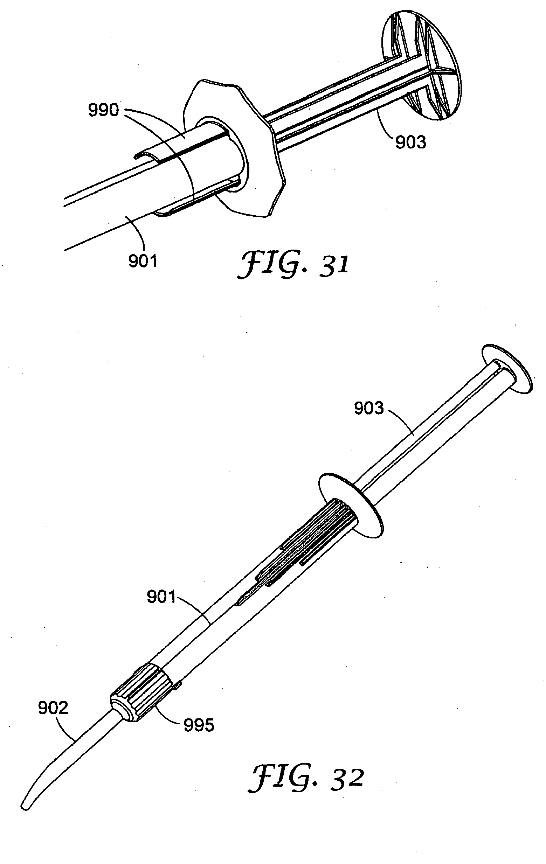

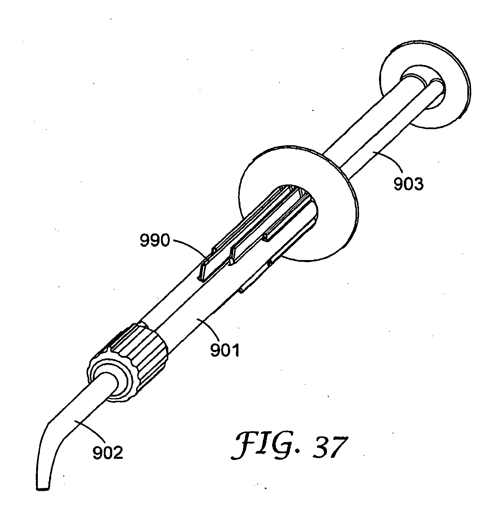

A further optional feature is shown in Figs. 31 and 37. In these embodiments, the outer surface of the cartridge is provided with a grip support facilitating the syringe to be held in any angle of rotation by the dentist. This feature is particularly preferred if the cartridge does not have a circular cross-sectional shape such as a twin barrel cartridge. In Fig. 31, the grip support is formed by two flanges projecting along the longitudinal axis of the cartridge from the rear end of the cartridge towards its front end thus providing a feeling to the dentist of having a cartridge with a circular cross-section in the hands. According to Fig. 37, the grip support is provided by a plurality of ribs or fins extending at the outer surface of the cartridge from the rear end of the cartridge a certain length along the longitudinal axis of the cartridge. The ribs have different heights in order to provide said feeling of holding a circular cartridge. In Fig. 37, the longest rib 990 has a larger height than the shorter ribs, the height decreasing from the longest rib 990 to the shortest rib because the longest rib is provided just in the groove between the two barrels of the cartridge 901.

A further preferred embodiment of this embodiment forming background information especially suitable for low viscosity materials is now described with reference to Figs. 32 to 36. The main difference to the embodiment shown, e.g., in Fig. 26 is the use of radially acting rotary slide valve 995 instead of an axially acting rotary slide valve. For low viscosity materials, a radially acting rotary slide valve is advantageous with regard to sealing of the cartridge during storage.

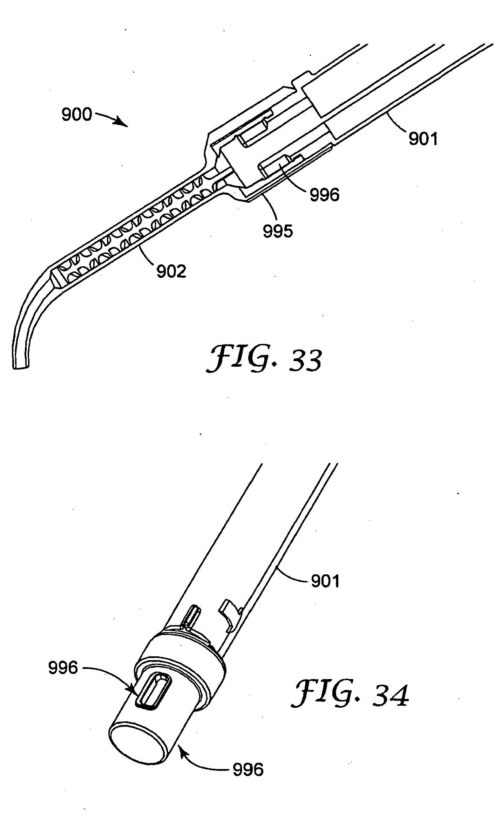

In the embodiment shown in Fig. 33 which is a cross-sectional view of syringe 900 with radially acting rotary slide valve 995, cartridge outlets 996 are arranged in a direction transverse to the axis of the syringe 900 thus providing the possibility of radially sealing the outlet bores of the syringe. As a result, a change of the flow direction of the materials will occur during application. The radial arrangement of the valve is advantageous for optimum sealing of the cartridge which is especially required for low viscosity materials. Opening and closing of the syringe 900 is made by rotating the mixing tip 902 by a predetermined angle. In the opened position, longitudinally arranged grooves within the coupling part of the mixing tip 902 are aligned with the radial outlet bores 996 in the cartridge 901 thus forming a passageway for the material components to flow into the mixing tip 902, as indicated in Fig. 33 by the arrows.

A perspective view of the front end of the cartridge 901 with outlet openings 996 is given in Fig. 34.

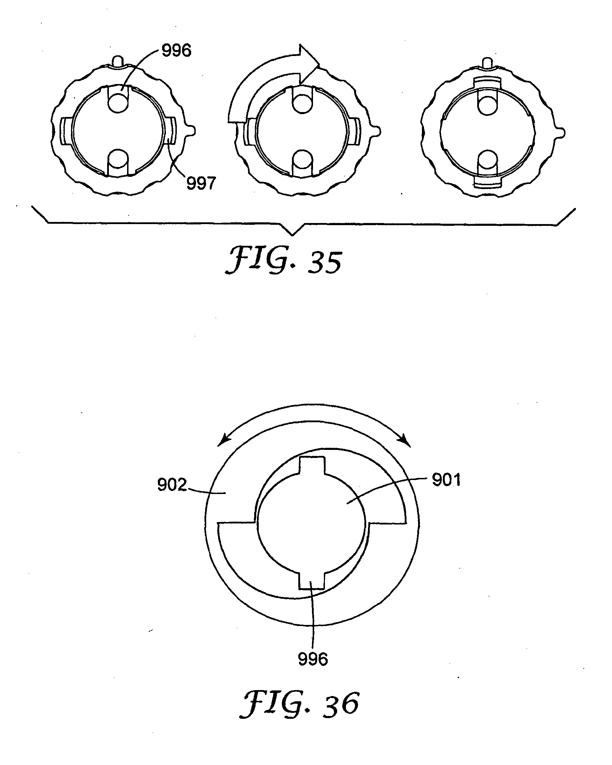

Fig. 35 shows a cross-sectional view through the openings 996 of the cartridge in three different rotational positions. In the left drawing, the syringe is closed. The openings 996 of the cartridge are directed vertically, whereas the corresponding passageways 997 in the valve are oriented horizontally, i.e. 90° offset from the outlets 996. Rotational movement of the mixing tip with the valve (in clockwise direction, for example, as shown in the centre drawing) brings the passageways 997 of the valve 995 into alignment with the openings 996.

Preferably, the valve 995 is provided with a "wedge function", as shown in schematic form in Fig. 36. Such "wedge function" provides a sealing pressure between the valve and the cartridge wall. In Fig. 36, reference numeral 902 denotes the cross-sectional area of the mixing tip, and reference numeral denotes the cartridge. Clockwise rotation of the cartridge relative to the mixing tip moves the outlet openings 996 until the cartridge abuts at the stepped configuration of the mixing tip. Rotation in the opposite direction causes a clamping and thus sealing between the cartridge and the mixing tip due to the wedged-shape.

## Claims

1. Unit-dose syringe (100) for a multi-component material, comprising:
- a cartridge (101) having a front end (107) and a rear end, and having a compartment (105, 106) for each component,
- a static mixer (112) connectable with said cartridge at its front end,
- a mixing tip (102) being integrally connected to the cartridge at said front end of said cartridge and receiving said static mixer, and
- a plunger (103) being located, in the inactivated state of the syringe, at said rear end of said cartridge, and movable towards the front end of said cartridge for dispensing material from said cartridge through said mixing tip, **characterized in that**
said static mixer comprises closure plugs (113) at its rear end for closing the outlet openings of said compartments of said cartridge, wherein said static mixer comprises a mixing helix (114), and wherein said static mixer comprises an outlet tip (115) at the front end of said mixing helix.

2. The syringe of claim 1, wherein said outlet tip is connected to said mixing helix by a hinge (116).

3. The syringe of claims 1 or 2, wherein said static mixer is collapsible.

4. The syringe of claim 1 or 2, wherein said outlet tip of said static mixer projects from the front end of said mixing tip when said static mixer is received in said mixing tip.

5. The syringe of claim 1, 2, or 3, wherein said outlet tip of said static mixer is accommodated within said mixing tip during storage of said syringe.

6. The syringe of claim 5, wherein said front end of said mixing tip and said outlet tip of said static mixer comprise corresponding retention means (119, 118) that allow said outlet tip to project beyond said front end of said mixing tip upon activation of said syringe but prevent that said outlet tip completely extends beyond said mixing tip.

7. The syringe of claim 6, wherein said retention means at said front end of said mixing tip comprises a recess (118) in the wall of said mixing tip, and said retention means at the outlet tip comprises a projection (119) at the circumference of the rear end of said outlet tip, said projection being engagable by said recess.

8. The syringe of any of claims 1 to 3, wherein said mixing tip forms an extension of a first of said compartments of said cartridge.

9. The syringe of any of claims 1 to 8, wherein said cartridge comprises at its outer surface extensions or protrusions being sized and shaped to provide said cartridge with a substantially circular circumferential outer surface.

10. The syringe of any of claims 1 to 9, wherein said cartridge has a rounded circumferential surface, and comprises at least one internal separation wall.

11. The syringe of any of claims 1 to 10, wherein said compartments are arranged concentrically.

12. The syringe of any of claims 1 to 11, wherein said plunger comprises a separate piston for each compartment of said cartridge.

13. The syringe of any of claims 1 to 12, wherein said cartridge is made from an elastic material.

14. The syringe of claim 13, wherein said elastic material is a thermoplastic elastomer.

15. The syringe of any of claims 1 to 14, wherein said plunger is made from a rigid material.

16. The syringe of any of claims 1 to 15, being pre-filled with a multi-component dental material.

## Patentansprüche

1. Einzeldosisspritze (100) für ein Mehrkomponentenmaterial, umfassend:
- eine Kartusche (101) mit einem vorderen Ende (107) und einem hinteren Ende und mit einem Behältnis (105, 106) für jede Komponente,
- einen statischen Mischer (112), der mit der Kartusche an ihrem vorderen Ende verbindbar ist,
- eine Mischspitze (102), die integral mit der Kartusche an dem vorderen Ende der Kartusche verbunden ist und den statischen Mischer aufnimmt, und
- einen Stößel (103), der sich im inaktivierten Zustand der Spritze an dem hinteren Ende der Kartusche befindet und in Richtung des vorderen Endes der Kartusche zur Abgabe von Material von der Kartusche durch die Mischspitze bewegbar ist,
**dadurch gekennzeichnet, dass**
der statische Mischer Verschlussstöpsel (113) an seinem hinteren Ende zum Verschließen der Auslassöffnungen der Behältnisse der Kartusche umfasst, wobei der statische Mischer eine Mischwendel (114) umfasst, und wobei der statische Mischer eine Auslassspitze (115) am vorderen Ende der Mischwendel umfasst.

2. Spritze nach Anspruch 1, wobei die Auslassspitze über ein Scharnier (116) mit der Mischwendel verbunden ist.

3. Spritze nach Anspruch 1 oder 2, wobei der statische Mischer kollabierbar ist.

4. Spritze nach Anspruch 1 oder 2, wobei die Auslassspitze des statischen Mischers von dem vorderen Ende der Mischspitze hervorsteht, wenn der statische Mischer in der Mischspitze aufgenommen ist.

5. Spritze nach Anspruch 1, 2 oder 3, wobei die Auslassspitze des statischen Mischers während der Lagerung der Spritze innerhalb der Mischspitze untergebracht ist.

6. Spritze nach Anspruch 5, wobei das vordere Ende der Mischspitze und die Auslassspitze des statischen Mischers entsprechende Haltemittel (119, 118) umfassen, die es der Auslassspitze gestatten, bei Aktivierung der Spritze über das vordere Ende der Mischspitze hinaus vorzustehen, aber die Auslassspitze daran hindern, sich gänzlich über die Mischspitze hinaus zu erstrecken.

7. Spritze nach Anspruch 6, wobei das Haltemittel am vorderen Ende der Mischspitze eine Vertiefung (118) in der Wand der Mischspitze umfasst und das Haltemittel an der Auslassspitze an dem Umfang des hinteren Endes der Auslassspitze einen Vorsprung (119) umfasst, wobei der Vorsprung mit der Vertiefung in Eingriff geraten kann.

8. Spritze nach einem der Ansprüche 1 bis 3, wobei die Mischspitze eine Erweiterung eines ersten der Behältnisse der Kartusche ausbildet.

9. Spritze nach einem der Ansprüche 1 bis 8, wobei die Kartusche an ihrer Außenfläche Erweiterungen oder Vorsprünge umfasst, die derart dimensioniert und gestaltet sind, dass sie der Kartusche eine im Wesentlichen kreisförmige Umfangsaußenfläche verleihen.

10. Spritze nach einem der Ansprüche 1 bis 9, wobei die Kartusche eine gerundete Umfangsfläche aufweist und mindestens eine innen liegende Trennungswand umfasst.

11. Spritze nach einem der Ansprüche 1 bis 10, wobei die Behältnisse konzentrisch angeordnet sind.

12. Spritze nach einem der Ansprüche 1 bis 11, wobei der Stößel einen separaten Kolben für jedes Behältnis der Kartusche umfasst.

13. Spritze nach einem der Ansprüche 1 bis 12, wobei die Kartusche aus einem elastischen Material hergestellt ist.

14. Spritze nach Anspruch 13, wobei es sich bei dem elastischen Material um ein thermoplastisches Elastomer handelt.

15. Spritze nach einem der Ansprüche 1 bis 14, wobei der Stößel aus einem steifen Material hergestellt ist.

16. Spritze nach einem der Ansprüche 1 bis 15, vorgefüllt mit einem zahnärztlichen Mehrkomponentenmaterial.

## Revendications

1. Seringue à dose unitaire (100) pour un produit à composants multiples, comprenant:
- une cartouche (101) présentant une extrémité avant (107) et une extrémité arrière, et comprenant un compartiment (105, 106) pour chaque composant;
- un mélangeur statique (112) qui peut être connecté à ladite cartouche à son extrémité avant;
- une pointe de mélange (102) qui est intégralement connectée à la cartouche à ladite extrémité avant de ladite cartouche et qui reçoit ledit mélangeur statique; et
- un plongeur (103) qui, à l'état non activé de la seringue, est situé à ladite extrémité arrière de ladite cartouche, et qui peut être déplacé en direction de l'extrémité avant de ladite cartouche pour distribuer un produit à partir de ladite cartouche à travers ladite pointe de mélange,
**caractérisé en ce que**:
ledit mélangeur statique comprend des bouchons de fermeture (113) à son extrémité arrière pour fermer les ouvertures de sortie desdits compartiments de ladite cartouche, dans laquelle ledit mélangeur statique comprend une hélice de mélange (114), et dans laquelle ledit mélangeur statique comporte une pointe de sortie (115) à l'extrémité avant de ladite hélice de mélange.

2. Seringue selon la revendication 1, dans laquelle ladite pointe de sortie est connectée à ladite hélice de mélange par une charnière (116).

3. Seringue selon revendication 1 ou 2, dans laquelle ledit mélangeur statique est pliable.

4. Seringue selon la revendication 1 ou 2, dans laquelle ladite pointe de sortie dudit mélangeur statique fait saillie à partir de l'extrémité avant de ladite pointe de mélange lorsque ledit mélangeur statique est reçu dans ladite pointe de mélange.

5. Seringue selon la revendication 1, 2 ou 3, dans laquelle ladite pointe de sortie dudit mélangeur statique est logée à l'intérieur de ladite pointe de mélange dans la condition de stockage de ladite seringue.

6. Seringue selon la revendication 5, dans laquelle ladite extrémité avant de ladite pointe de mélange et ladite pointe de sortie dudit mélangeur statique comprennent des moyens de retenue correspondants (119, 118) qui permettent à ladite pointe de sortie de faire saillie au-delà de ladite extrémité avant de ladite pointe de mélange au moment de l'actionnement de ladite seringue, mais qui empêchent que ladite pointe de sortie s'étende complètement au-delà de ladite pointe de mélange.

7. Seringue selon la revendication 6, dans laquelle lesdits moyens de retenue à ladite extrémité avant de ladite pointe de mélange présentent un évidement (118) dans la paroi de ladite pointe de mélange, et lesdits moyens de retenue à la pointe de sortie comportent une saillie (119) à la circonférence de l'extrémité arrière de ladite pointe de sortie, ladite saillie pouvant être engagée par ledit évidement.

8. Seringue selon l'une quelconque des revendications 1 à 3, dans laquelle ladite pointe de mélange forme une extension d'un premier desdits compartiments de ladite cartouche.

9. Seringue selon l'une quelconque des revendications 1 à 8, dans laquelle ladite cartouche comporte à sa surface extérieure des extensions ou des saillies qui sont dimensionnées et configurées de manière à doter ladite cartouche d'une surface extérieure circonférentielle sensiblement circulaire.

10. Seringue selon l'une quelconque des revendications 1 à 9, dans laquelle ladite cartouche présente une surface circonférentielle arrondie, et comprend au moins une paroi de séparation interne.

11. Seringue selon l'une quelconque des revendications 1 à 10, dans laquelle lesdits compartiments sont agencés de façon concentrique.

12. Seringue selon l'une quelconque des revendications 1 à 11, dans laquelle ledit plongeur comprend un piston séparé pour chaque compartiment de ladite cartouche.

13. Seringue selon l'une quelconque des revendications 1 à 12, dans laquelle ladite cartouche est constituée d'une matière élastique.

14. Seringue selon la revendication 13, dans laquelle ladite matière élastique est un élastomère thermoplastique.

15. Seringue selon l'une quelconque des revendications 1 à 14, dans laquelle ledit plongeur est constitué d'un matériau rigide.

16. Seringue selon l'une quelconque des revendications 1 à 15, ladite seringue étant pré-remplie d'un produit dentaire à composants multiples.
